(19) Europäisches Patentamt
European Patent Office
Office européen des brevets

(11) Numéro de publication: **0 294 295 B1**

(12) **FASCICULE DE BREVET EUROPEEN**

(45) Date de publication de fascicule du brevet: **26.08.92**

(51) Int. Cl.[5]: **C07D 207/48**, A61K 31/40

(21) Numéro de dépôt: **88401355.8**

(22) Date de dépôt: **03.06.88**

Le dossier contient des informations techniques présentées postérieurement au dépôt de la demande et ne figurant pas dans le présent fascicule.

# (54) Dérivés de la 1-arylsulfonyl 2-oxo 5-alcoxy pyrrolidine, leur procédé de préparation, leur application comme médicaments et les compositions les renfermant.

(30) Priorité: **05.06.87 IT 2080987**

(43) Date de publication de la demande:
**07.12.88 Bulletin 88/49**

(45) Mention de la délivrance du brevet:
**26.08.92 Bulletin 92/35**

(84) Etats contractants désignés:
**CH DE ES FR GB GR IT LI NL**

(56) Documents cités:
**EP-A- 0 138 721**

**THE JOURNAL OF ORGANIC CHEMISTRY, vol. 31, N 9, septembre 1966, pages 2847-2853; J.E. FRANZ et al.: "Reactions of sulfonyl azides and sulfonamides with vinyl ethers"**

(73) Titulaire: **ROUSSEL-UCLAF**
**35, Boulevard des Invalides**
**F-75007 Paris(FR)**

(72) Inventeur: **Toja, Emilio**
**Via Plezzo, 80**
**Milano(IT)**
Inventeur: **Gorini, Carlo**
**Via Orcagna 4**
**Milano(IT)**
Inventeur: **Barzaghi, Fernando**
**Via Monteverdi 21**
**I-20052 Monza-Milan(IT)**
Inventeur: **Galliani, Giulio**
**13, Via Silva**
**1 Monza (MI)(IT)**

(74) Mandataire: **Tonnellier, Marie-José et al**
**111, route de Noisy B.P. no 9**
**F-93230 Romainville(FR)**

**EP 0 294 295 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne de nouveaux dérivés de la 1-arylsulfonyl 2-oxo 5-alcoxy pyrrolidine, leur procédé de préparation et leur application comme médicaments.

On connaissait déjà des 4-hydroxy, 4-alkloxy et 4-acyloxy-1-benzenesulfonyl-2-oxopyrrolidines (cf EP-A-138721) utilisables dans le traitement des troubles de la mémoire ou du surmenage intellectuel.

On vient de découvrir des composés apparentés présentant des propriétés biologiques intéressantes.

L'invention a pour objet les composés de formule générale (I) :

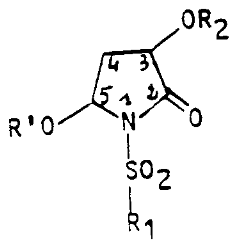

(I)

dans laquelle R' et $R_2$, identiques ou différents, représentent un atome d'hydrogène, un radical alcoyle, linéaire, ramifié ou cyclique, renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle, $R_1$ représente un radical phényle ou naphtyle, un radical thiényle, furyle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b] pyranyle, benzoxazolyle ou morpholinyle, le radical $R_1$ étant éventuellement substitué par un ou plusieurs substituants choisis dans le groupe constitué par le radical hydroxyle, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogènes, les groupements $CF_3$, $SCF_3$, $OCF_3$, $NO_2$, $NH_2$ ou C≡N, le radical phényle et les groupements alcoylsulfonyles renfermant de 1 à 6 atomes de carbone.

Les composés de formule (I) peuvent exister sous différentes formes diastéréoisomères et l'invention a pour objet ces différentes formes diastéréoisomères ainsi que leurs mélanges.

Par radical alcoyle, on entend de préférence un radical renfermant de 1 à 6 atomes de carbone, par exemple, le radical méthyle, éthyle, n-propyle, isopropyle, n-butyle, isobutyle, terbutyle, n-pentyle, n-hexyle, cyclopropyle, cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

Lorsque le radical $R_1$ est substitué par un ou plusieurs radicaux alcoyles il s'agit de préférence d'un ou plusieurs radicaux méthyle, éthyle, isopropyle, éthényle ou éthynyle.

Lorsque le radical $R_1$ est substitué par un ou plusieurs atomes d'halogène, il s'agit de préférence d'un ou de plusieurs atomes de fluor, de chlore ou de brome.

Parmi les composés préférés de l'invention, on peut citer :

- les composés de formule (I) dans lesquels $R_2$ représente un atome d'hydrogène,
- les composés de formule (I) dans lesquels $R_1$ représente un radical phényle,
- les composés de formule (I) dans lesquels R' représente un radical alcoyle linéaire, ramifié ou cyclique, renfermant jusqu'à 4 atomes de carbone et tout particulièrement :
- la 1-benzènesulfonyl 2-oxo 3-hydroxy 5-éthoxy pyrrolidine.

L'invention a également pour objet un procédé de préparation des composés de formule (I), caractérisé en ce que l'on soumet un composé de formule (II) :

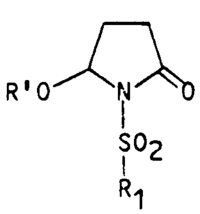

(II)

dans laquelle $R_1$ et $R'$ conservent leur signification précédente, à l'action d'un agent d'hydroxylation, pour obtenir le composé de formule ($I_A$) correspondant :

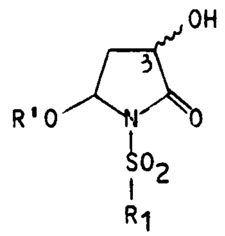

($I_A$)

sous forme d'un mélange d'isomères en 3 que l'on sépare, si désiré, en chacun des isomères, puis soumet, si désiré, le mélange d'isomères ($I_A$) ou chacun de ses constituants à l'action d'un agent d'éthérification ou d'estérification pour obtenir le composé de formule ($I_B$) :

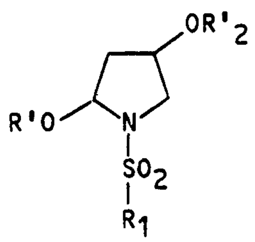

($I_B$)

dans laquelle $R'_2$ a les valeurs indiquées précédemment pour $R_2$, à l'exception d'un atome d'hydrogène, composé de formule ($I_B$) que l'on sépare, le cas échéant, en chacun de ses isomères.

Dans un mode de réalisation préférée du procédé de l'invention, le réactif d'hydroxylation est l'oxodiperoxy molibdeno pyridine hexaméthyl phosphororamide ou $MoO_5$.Py.HMPA et l'on opère en présence de diisopropylamidure de lithium au sein d'un mélange de tétrahydrofuranne et d'hexane ou la 2-sulfonyloxaziridine et l'on opère en présence de bis-triméthylsilyl amidure de lithium au sein d'un mélange de tétrahydrofuranne et d'hexane.

Les isomères en position 3 sont séparés par les méthodes classiques de chromatographie et de cristallisation.

Les réactions d'éthérification et d'estérification sont réalisées selon les procédés classiques.

Les composés de l'invention présentent d'intéressantes propriétés pharmacologiques ; ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicament, le produit de l'exemple 1.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 1 administré par voie orale.

La présente invention a également pour objet les compositions pharmaceutiques renfermant comme principe actif au moins un produit de formule (I).

Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) utilisés comme produits de départ sont des produits décrits et revendiqués dans la demande de brevet européen publiée sous le n¤ 0 229 566 déposée le 23 décembre 1986.

Les exemples suivants illustrent l'invention sans toutefois la limiter.

**Exemple 1 : 1-benzènesulfonyl 2-oxo 3-hydroxy 5-éthoxy pyrrolidine.**

A 58 cm3 d'une solution de lithium diisopropylamide (0,68 M) dans l'hexane-THF (1-1), on ajoute à -70¤C 4,83 g de 5-éthoxy 1-benzènesulfonyl 2-pyrrolidinone en solution dans 100 cm3 de tétrahydrofuranne. On maintient la température à -70¤C pendant 15 minutes puis ajoute, en une fois, à -60¤C 11,68 g du complexe oxodiperoxymolybdenium pyridine hexaméthyl phosphoramide MoOPH (MoOPH : oxodiperoxymolybdenium pyridine hexaméthyl phosphotriamide $MoO_5$. Py.HMPA préparé selon VEDEJS, ENGLER, TELSCHOW J. ORG. CHEM. (1978), 43, 188), maintient encore à -50¤C pendant 2 heures puis pendant 45 minutes à -5¤C. On décompose le milieu réactionnel en ajoutant à -45¤C 80 cm3 d'une solution de sulfite de sodium, le milieu est ensuite relargué à 20¤C avec 80 cm3 d'une solution saturée de chlorure de sodium. On extrait avec du chloroforme, sèche et évapore à sec. On chromatographie sur silice (éluant : hexane-acétate d'éthyle 1-3) et obtient 0,74 g de mélange des 2 isomères. F = 80-98¤C.

| Analyse : $C_{12}H_{15}NO_5S$ | | | |
|---|---|---|---|
| Calculé : | C% 50,51 | H% 5,30 | N% 4,91 |
| Trouvé : | 50,8 | 5,27 | 4,82 |

On obtient l'un des isomères par cristallisation fractionnée dans un mélange isopropanol et éther isopropylique. F = 95-97¤C.

Le second isomère est obtenu par chromatographie sur silice (éluant : hexane-acétate d'éthyle 1-2). F = 128-130¤C recristallisé de l'isopropanol. L'analyse HPLC du mélange a mis en évidence les 2 isomères dans un rapport de 1 pour 1 (colonne 5 microns Lichrosorb Si 60 Merck 250 mm x 4 mm, phase mobile : tétrahydrofuranne à 1% acide acétique-hexane 30-70. Elution 1 ml par minute. Injection 20 microlitres, révélation UV 260 nanomètres).

Comme alternative et plus avantageusement, on peut séparer les 2 isomères par chromatographie sur colonne "Lobar Lichroprep Si 60 (Merck), élution avec tétrahydrofuranne à 1% acide acétique-hexane 30-70. L'isomère qui fond à 128-130¤C a un Rf. de 0,16 et l'isomère qui fond à 95-97¤C a un Rf. de 0,11.

**Préparation du 1-benzènesulfonyl 2-oxo 5-éthoxy pyrrolidine.**

A 45 g de 5-éthoxy pyrrolidin-2-one en solution dans 140 cm3 de tétrahydrofuranne, on ajoute 21,8 cm3 d'une solution 1,6 M de butyllithium dans l'hexane refroidie à -10¤C. Après 45 minutes, on ajoute 6,15 g de chlorure de benzènesulfonyle dans le tétrahydrofuranne, maintient à -10¤C pendant 2 heures puis concentre sous pression réduite et reprend avec de l'éthanol. On isole le produit par filtration et obtient 2,8 g de produit attendu. F = 112-113¤C cristallisé de l'isopropanol.

| Analyse : | | | |
|---|---|---|---|
| Calculé : | C% 53,52 | H% 5,61 | N% 5,20 |
| Trouvé : | 53,30 | 5,64 | 5,10 |

**Exemple 2 : 1-benzènesulfonyl 2-oxo 3-hydroxy 5-éthoxy pyrrolidine.**

Dans une solution de 10 g de 5-éthoxy 1-benzènesulfonyl pyrrolidin-2-one dans 300 cm3 de tétrahydrofuranne anhydre, refroidie à -78¤C, on laisse couler goutte à goutte 0,037 moles d'une solution de bis-triméthylsilyl amidure de lithium dans le mélange hexane-tétrahydrofuranne (1-1) en opérant à -78¤C. On agite pendant 1 heure à -78¤C puis ajoute 14,55 g de 2-sulfonyl-oxaziridine. On agite à -78¤C pendant 2 heures, laisse revenir à -5¤C, refroidit encore à -30¤C puis ajoute 130 cm3 d'une solution aqueuse saturée de chlorure d'ammonium en maintenant la température à -30¤C environ. On laisse revenir à 20¤C et ajoute 130 cm3 d'une solution aqueuse saturée de chlorure de sodium. On sépare la phase organique et on extrait la phase aqueuse au chloroforme. On réunit les phases organiques, les sèche, évapore le solvant sous

pression réduite et chromatographie le résidu sur silice en éluant au mélange acétate d'éthyle-hexane (2-1). On reprend à l'éther isopropylique et obtient 250 g de produit attendu. F = 108-116¤C. (mélange d'isomères cis et trans dans lequel l'isomère trans est prédominant).

**Exemple 3 : 1-benzènesulfonyl 2-oxo 3-hydroxy 5-isopropyloxy pyrrolidine.**

Dans une solution de 3,00 g de 1-benzènesulfonyl 5-isopropyloxy 2-pyrrolidinone (décrite dans la demande européenne 229 566) dans 60 cm3 de tétrahydrofuranne anhydre, refroidie à -78¤C, on laisse couler goutte à goutte, sous courante d'azote, 36,39 cm3 d'une solution 0,64M de diisopropylamidure de lithium dans le mélange tétrahydrofuranne-hexane (1-1) en opérant à -78¤C. On agite à -78¤C pendant 45 minutes, puis ajoute, toujours à -78¤C, 6,90 g d'oxodipéroxymolybdénium pyridine hexaméthylphosphorotriamide. On laisse revenir le mélange à -5¤C, refroidit de nouveau à environ -50¤C, agite pendant 1 heure, puis à -18¤C pendant 2 heures. On refroidit à -50¤C environ et laisse couler goutte à goutte 50 cm3 d'une solution aqueuse saturée de chlorure d'ammonium. On laisse revenir à température ambiante et laisse couler goutte à goutte 50 cm3 d'une solution aqueuse saturée de chlorure de sodium. On sépare la phase organique et extrait la phase aqueuse au chloroforme. Les phases organiques réunies sont lavées à l'acide chlorhydrique 2N. On sèche, évapore le solvant sous pression réduite et chromatographie le résidu sur silice en éluant au mélange acétate d'ethyle-hexane (1-1). On cristallise dans l'isopropanol et obtient 0,40 g de solide blanc. F = 127-129¤C (isomère trans).

| Analyse : $C_{13}H_{17}NO_5S$ = 299,35 | | | |
|---|---|---|---|
| Calculé :<br>Trouvé : | C% 52,16<br>52,35 | H% 5,72<br>5,65 | N%4,68<br>4,61 |

**Exemple 4 : 1-benzènesulfonyl 2-oxo 3-éthoxy 5-éthoxypyrrolidine.**

A un mélange formé par 1 g de 1-benzènesulfonyl 2-oxo 3-hydroxy 5-éthoxy pyrrolidine (isomère trans), 30 cm3 de chlorure de méthylène anhydre et une petite quantité de chlorure d'aluminium, on ajoute goutte à goutte à -10¤C, 5 cm3 de diazoéthane. On rajoute un peu de chlorure d'aluminium (quantité catalytique) et rajoute 6 $cm^3$ environ de diazoéthane, toujours à -10¤C. Après une heure, on laisse revenir à température ambiante, lave avec 10 cm3 d'acide acétique à 1% puis avec $NaHCl_3$ à 5%, sèche, concentre à sec sous pression réduite et chromatographie sur silice en éluant avec le mélange acétate d'éthyle-hexane (1-1). On obtient 0,17 g de produit attendu.

| Analyse : $C_{14}H_{19}NO_5S$ = 313,376 | | | |
|---|---|---|---|
| Calculé :<br>Trouvé : | C% 53,66<br>53,92 | H% 6,11<br>5,97 | N% 4,47<br>4,34 |

**Exemple 5 : 1-benzènesulfonyl 2-oxo 3-acétoxy 5-éthoxy pyrrolidine.**

Une solution de 0,50 g de 1-benzènesulfonyl 2-oxo 3-hydroxy 5-éthoxy pyrrolidine (isomère trans) dans 10 cm3 d'anhydride acétique est chauffée à l'ébullition pendant 1 heure. On laisse refroidir à température ambiante et on évapore l'anhydride acétique. On reprend avec du toluène et évapore l'azéotrope sous pression réduite. On chromatographie le résidu sur silice en éluant au mélange acétate d'éthyle-hexane (1-1). On obtient 0,48 g de produit attendu.

**Exemple 6 : Compositions pharmaceutiques.**

a) On a préparé des comprimés répondant à la formule suivante :
- Produit de l'exemple 1 (mélange d'isomères) 100 mg
- Excipient q.s. pour un comprimé terminé à 300 mg.

(Détail de l'excipient : lactose, amidon de blé, amidon traité, amidon de riz, stéarate de magnésium, talc).

b) On a préparé des gélules répondant à la formule suivante :

- Produit de l'exemple 1 (mélange d'isomères) 200 mg
- Excipient q.s. pour une gélule terminée à 300 mg.

(Détail de l'excipient : talc, stéarate de magnésium, Aerosil®).

## ETUDE PHARMACOLOGIOUE

### Toxicité aiguë et comportement des produits de l'invention.

Nous avons utilisé des souris mâles ($CD_1$ Charles Rivers) d'un poids de 22-23 g à jeun depuis 16 heures. Les produits leur furent administrés normalement par voie orale aux doses de 1000 - 500 - 250 mg/kg.

L'effet des produits sur le comportement des animaux fut évalué suivant la méthode décrite par Irvin (Psychopharmacologia (1968), 13, 222-257) durant les 8 premières heures et à la 24ème heure.

La mortalité fut relevée pendant les 7 jours suivant le traitement.

La $DL_{50}$ a ainsi été trouvée supérieure à 1000 mg/kg.

### Apprentissage et mémorisation.

Nous avons utilisé des souris mâles ($CD_1$ Charles Rivers) d'un poids de 25-30 g. Les animaux sont placés dans la partie lumineuse d'un box à deux compartiments communicants par une ouverture (G. Galliani, R. Cesana and F. Barzaghi, Med. Sci. Res. (1987), 15, 313-314.

A l'instant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on la remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale aux doses de 12,5 ; 25 ; 50 ; 100 et 200 mg/kg.

Nous avons utilisé de 10 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence.

Les résultats sont exprimés en pourcentages d'augmentation du temps de latence par rapport aux témoins correspondants. Des résultats obtenus avec 2 produits de référence sont fournis.

Les résultats sont les suivants :

**Pourcentage d'augmentation du temps de latence par rapport aux témoins**

| | Dose mg/kg os | | | |
|---|---|---|---|---|
| | 200 | 100 | 50 | 25 |
| Produit de l'exemple 1 (mélange) | 103* | 84* | 65* | 21 |
| Isomère F. 128-130°C | 110* | 106* | 52* | 22 |
| Isomère F. 95-97°C | 121* | 112 | 63* | 38 |
| PIRACETAM | 20 | 48* | 10 | 19 |
| ANIRACETAM | 32 | 88* | 77* | 39 |

* Valeurs statistiquement différentes par rapport aux témoins.

**Conclusion :**

Le produit de l'exemple 1 et ses isomères ont montré une activité antiamnésique importante à des doses comprises entre 50 et 200 mg/kg per os et très supérieure à celle des produits de référence.

**Revendications**

**Revendications pour les Etats contractants suivants : CH, DE, FR, GB, IT, LI, NL**

**1.** Les composés de formule générale (I) :

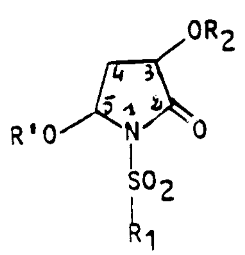

(I)

dans laquelle R' et $R_2$, identiques ou différents, représentent un atome d'hydrogène, un radical alcoyle, linéaire, ramifié ou cyclique, renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle, $R_1$ représente un radical phényle ou naphtyle, un radical thiényle, furyle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b] pyranyle, benzoxazolyle ou morpholinyle, le radical $R_1$ étant éventuellement substitué par un ou

plusieurs substituants choisis dans le groupe constitué par le radical hydroxyle, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogènes, les groupements $CF_3$, $SCF_3$, $OCF_3$, $NO_2$, $NH_2$ ou C≡N, le radical phényle et les groupements alcoylsulfonyles renfermant de 1 à 6 atomes de carbone.

**2.** Les composés de formule (I) tels que définis à la revendication 1 dans lesquels $R_2$ représente un atome d'hydrogène.

**3.** Les composés de formule (I) tels que définis à la revendication 1 ou 2 dans lesquels $R_1$ représente un radical phényle.

**4.** Les composés de formule (I) tels que définis à l'une quelconque des revendications 1 à 3 dans lesquels R' représente un radical alcoyle linéaire, ramifié ou cyclique, renfermant jusqu'à 4 atomes de carbone.

**5.** Le produit défini à la revendication 1 dont le nom suit :
- la 1-benzènesulfonyl 2-oxo 3-hydroxy 5-éthoxy pyrrolidine.

**6.** A titre de médicaments, les composés de formule (I) tels que définis à l'une quelconque des revendications 1 à 4.

**7.** A titre de médicament, le composé de la revendication 5.

**8.** Les compositions pharmaceutiques renfermant comme principe actif au moins un médicament défini à la revendication 6 ou 7.

**9.** Procédé de préparation des composés de formule (I) tels que définis à la revendication 1, caractérisé en ce que l'on soumet un composé de formule (II) :

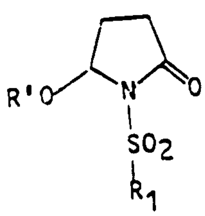

(II)

dans laquelle $R_1$ et R' conservent leur signification précédente, à l'action d'un agent d'hydroxylation, pour obtenir le composé de formule ($I_A$) correspondant :

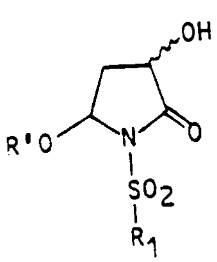

($I_A$)

sous forme d'un mélange d'isomères en 3 que l'on sépare, si désiré, en chacun des isomères, puis soumet, si désiré, le mélange d'isomères ($I_A$) ou chacun de ses constituants à l'action d'un agent d'éthérification ou d'estérification pour obtenir le composé de formule ($I_B$) :

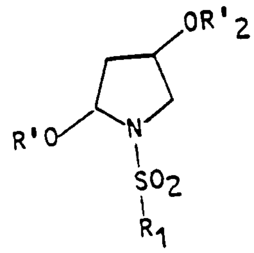

$(I_B)$

dans laquelle $R'_2$ a les valeurs indiquées précédemment pour $R_2$, à l'exception d'un atome d'hydrogène, composé de formule $(I_B)$ que l'on sépare, le cas échéant, en chacun de ses isomères.

**Revendications pour l'Etat contractant suivant : ES**

**1.** Procédé pour préparer des composés de formule générale :

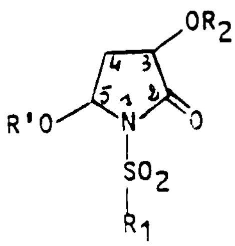

(I)

dans laquelle R' et $R_2$, identiques ou différents, représentent un atome d'hydrogène, un radical alcoyle, linéaire, ramifié ou cyclique, renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle, $R_1$ représente un radical phényle ou naphtyle, un radical thiényle, furyle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b] pyranyle, benzoxazolyle ou morpholinyle, le radical $R_1$ étant éventuellement substitué par un ou plusieurs substituants choisis dans le groupe constitué par le radical hydroxyle, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogènes, les groupements $CF_3$, $SCF_3$, $OCF_3$, $NO_2$, $NH_2$ ou C≡N, le radical phényle et les groupements alcoylsulfonyles renfermant de 1 à 6 atomes de carbone caractérisé en ce que l'on soumet un composé de formule (II) :

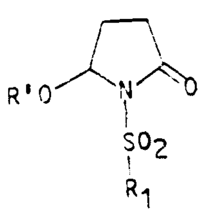

(II)

dans laquelle $R_1$ et R' conservent leur signification précédente, à l'action d'un agent d'hydroxylation, pour obtenir le composé de formule $(I_A)$ correspondant :

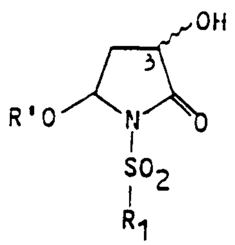

$(I_A)$

sous forme d'un mélange d'isomères en 3 que l'on sépare, si désiré, en chacun des isomères, puis soumet, si désiré, le mélange d'isomères $(I_A)$ ou chacun de ses constituants à l'action d'un agent d'éthérification ou d'estérification pour obtenir le composé de formule $(I_B)$ :

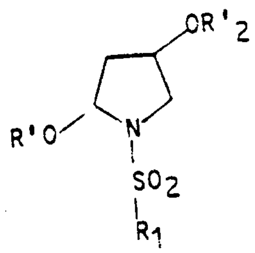

$(I_B)$

dans laquelle $R'_2$ a les valeurs indiquées précédemment pour $R_2$, à l'exception d'un atome d'hydrogène, composé de formule $(I_B)$ que l'on sépare, le cas échéant, en chacun de ses isomères.

**2.** Procédé selon la revendication 1, caractérisé en ce que l'on utilise au départ un composé de formule (II) dans laquelle $R_1$ représente un radical phényle.

**3.** Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 4 atomes de carbone.

**4.** Procédé selon l'une quelconque des revendications 1 à 3, caractérisé en ce que l'on prépare des composés de formule (I) dans laquelle $R_2$ représente un atome d'hydrogène.

**5.** Procédé selon l'une quelconque des revendications 1 à 4, caractérisé en ce que l'on prépare la 1-benzène sulfonyl 2-oxo 3-hydroxy 5-éthoxy pyrrolidine.

**Revendications pour l'Etat contractant suivant : GR**

**1.** Procédé pour préparer des composés de formule générale :

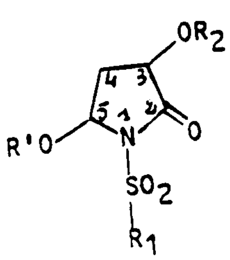

(I)

dans laquelle R' et $R_2$, identiques ou différents, représentent un atome d'hydrogène, un radical alcoyle, linéaire, ramifié ou cyclique, renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle, $R_1$ représente un radical phényle ou naphtyle, un radical thiényle, furyle,

pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b] pyranyle, benzoxazolyle ou morpholinyle, le radical $R_1$ étant éventuellement substitué par un ou plusieurs substituants choisis dans le groupe constitué par le radical hydroxyle, le radical acétoxy, le radical méthoxy ou le radical benzyloxy, les radicaux alcoyles linéaires, ramifiés ou cycliques, saturés ou insaturés, comportant jusqu'à 18 atomes de carbone, les atomes d'halogènes, les groupements $CF_3$, $SCF_3$, $OCF_3$, $NO_2$, $NH_2$ ou C≡N, le radical phényle et les groupements alcoylsulfonyles renfermant de 1 à 6 atomes de carbone caractérisé en ce que l'on soumet un composé de formule (II) :

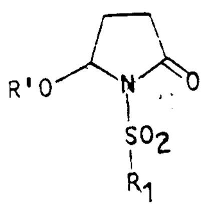

(II)

dans laquelle $R_1$ et R' conservent leur signification précédente, à l'action d'un agent d'hydroxylation, pour obtenir le composé de formule ($I_A$) correspondant :

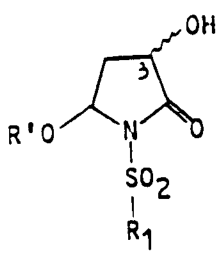

($I_A$)

sous forme d'un mélange d'isomères en 3 que l'on sépare, si désiré, en chacun des isomères, puis soumet, si désiré, le mélange d'isomères ($I_A$) ou chacun de ses constituants à l'action d'un agent d'éthérification ou d'estérification pour obtenir le composé de formule ($I_B$) :

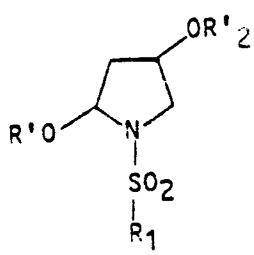

($I_B$)

dans laquelle $R'_2$ a les valeurs indiquées précédemment pour $R_2$, à l'exception d'un atome d'hydrogène, composé de formule ($I_B$) que l'on sépare, le cas échéant, en chacun de ses isomères.

2. Procédé selon la revendication 1, caractérisé en ce que l'on utilise au départ un composé de formule (II) dans laquelle $R_1$ représente un radical phényle.

3. Procédé selon la revendication 1 ou 2, caractérisé en ce que l'on utilise au départ un composé de formule (II) dans laquelle R' représente un radical alcoyle linéaire, ramifié ou cyclique, renfermant jusqu'à 4 atomes de carbone.

4. Procédé selon l'une quelconque des revendications 1 à 3, caractérisé en ce que l'on prépare des composés de formule (I) dans laquelle $R_2$ représente un atome d'hydrogène.

5. Procédé selon l'une quelconque des revendications 1 à 4, caractérisé en ce que l'on prépare la 1-benzène sulfonyl 2-oxo 3-hydroxy 5-éthoxy pyrrolidine.

6. Procédé de préparation de compositions pharmaceutiques, caractérisé en ce que l'on met, à titre de principe actif, l'un au moins des produits de formule (I), telle que définie à la revendication 1, sous une forme destinée à cet usage.

7. Procédé de préparation de compositions pharmaceutiques, caractérisé en ce que l'on met, à titre de principe actif, l'un au moins des produits de formule (I), telle que définie à l'une quelconque des revendications 2 à 4, sous une forme destinée à cet usage.

8. Procédé de préparation de compositions pharmaceutiques, caractérisé en ce que l'on met, à titre de principe actif, la benzènesulfonyl 2-oxo 3-hydroxy 5-éthoxy pyrrolodine, sous une forme destinée à cet usage.

**Claims**

**Claims for the following Contracting States : CH, DE, FR, GB, IT, LI, NL**

1. The compounds of general formula (I):

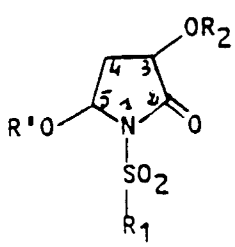

(I)

in which R' and $R_2$, identical or different, represent a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical, $R_1$ represents a phenyl or naphthyl radical, or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the $R_1$ radical being optionally substituted by one or more substituents chosen from the group constituted by the hydroxyl radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical and alkylsulphonyl groups containing 1 to 6 carbon atoms.

2. The compounds of formula (I) as defined in claim 1 in which $R_2$ represents a hydrogen atom.

3. The compounds of formula (I) as defined in claim 1 or 2 in which $R_1$ represents a phenyl radical.

4. The compounds of formula (I) as defined in any one of claims 1 to 3 in which R' represents a linear, branched or cyclic alkyl radical containing up to 4 carbon atoms.

5. The product defined in claim 1 of which the name follows:
   - 1-benzenesulphonyl-2-oxo-3-hydroxy-5-ethoxy pyrrolidine.

6. As medicaments, the compounds of formula (I) as defined in any one of claims 1 to 4.

7. As a medicament, the compound of claim 5.

8. The pharmaceutical compositions containing as active ingredient at least one medicament defined in claim 6 or 7.

**9.** Preparation process for compounds of formula (I) as defined in claim 1, characterized in that a compound of formula (II):

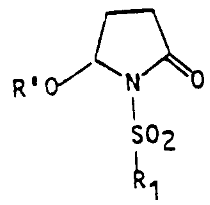

(II)

in which $R_1$ and R' keep their previous meaning, is subjected to the action of a hydroxylation agent, in order to obtain the corresponding compound of formula ($I_A$):

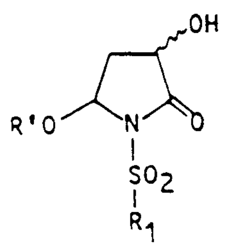

($I_A$)

in the form of a mixture of isomers in position 3 which is separated, if desired, into each of the isomers, then if desired, the isomer mixture ($I_A$) or each of its constituents is subjected to the action of an etherification or esterification agent in order to obtain the compound of formula ($I_B$):

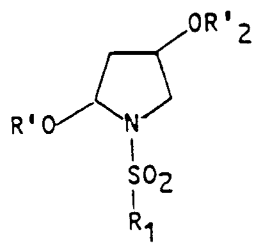

($I_B$)

in which $R'_2$ has the values indicated previously for $R_2$, with the exception of a hydrogen atom, which compound of formula ($I_B$) is separated, if appropriate, into each of its isomers.

**Claims for the following Contracting State : ES**

**1.** Process for the preparation of compounds of general formula:

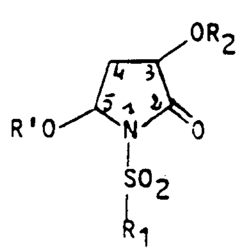

(I)

in which R' and $R_2$, identical or different, represent a hydrogen atom, a linear, branched or cyclic alkyl radical containing up to 8 carbon atoms, an alkenyl radical containing 2 to 8 carbon atoms, an acyl

radical containing 1 to 6 carbon atoms or a benzyl or phenethyl radical, $R_1$ represents a phenyl or naphthyl radical, or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the $R_1$ radical being optionally substituted by one or more substituents chosen from the group constituted by the hydroxyl radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical and alkylsulphonyl groups containing 1 to 6 carbon atoms, characterized in that a compound of formula (II):

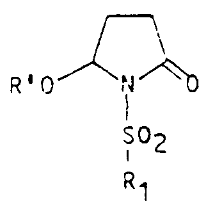

(II)

in which $R_1$ and R' keep their previous meaning, is subjected to the action of a hydroxylation agent, in order to obtain the corresponding compound of formula ($I_A$):

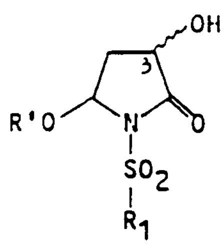

($I_A$)

in the form of a mixture of isomers in position 3 which is separated, if desired, into each of the isomers, then if desired, the isomer mixture ($I_A$) or each of its constituents is subjected to the action of an etherification or esterification agent in order to obtain the compound of formula ($I_B$):

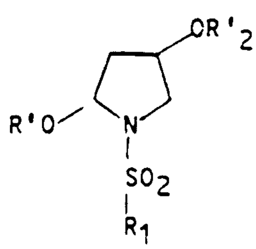

($I_B$)

in which $R'_2$ has the values indicated previously for $R_2$, with the exception of a hydrogen atom, which compound of formula ($I_B$) is separated, if appropriate, into each of its isomers.

2. Process according to claim 1, characterized in that at the start a compound of formula (II) is used in which $R_1$ represents a phenyl radical.

3. Process according to claim 1 or 2, characterized in that at the start a compound of formula (II) is used in which R' represents a linear, branched or cyclic alkyl radical, containing up to 4 carbon atoms.

4. Process according to any one of claims 1 to 3, characterized in that compounds of formula (I) are prepared in which $R_2$ represents a hydrogen atom.

**5.** Process according to any one of claims 1 to 4, characterized in that 1-benzene-sulphonyl-2-oxo-3-hydroxy-5-ethoxy pyrrolidine is prepared.

**Claims for the following Contracting State : GR**

**1.** Process for the preparation of compounds of general formula:

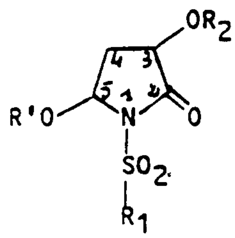

(I)

in which R' and $R_2$, identical or different, represent a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical, $R_1$ represents a phenyl or naphthyl radical, or one of the following radicals: thienyl, furyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl, the $R_1$ radical being optionally substituted by one or more substituents chosen from the group constituted by the hydroxyl radical, the acetoxy radical, the methoxy radical or the benzyloxy radical, saturated or unsaturated linear, branched or cyclic alkyl radicals containing up to 18 carbon atoms, halogen atoms, $CF_3$, $SCF_3$, $OCF_3$, $NO_2$, $NH_2$ or C≡N groups, the phenyl radical and alkylsulphonyl groups containing 1 to 6 carbon atoms, characterized in that a compound of formula (II):

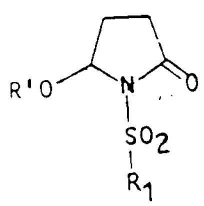

(II)

in which $R_1$ and R' keep their previous meaning, is subjected to the action of a hydroxylation agent, in order to obtain the corresponding compound of formula ($I_A$):

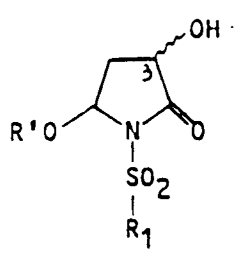

($I_A$)

in the form of a mixture of isomers in position 3 which is separated, if desired, into each of the isomers, then if desired, the isomer mixture ($I_A$) or each of its constituents is subjected to the action of an etherification or esterification agent in order to obtain the compound of formula ($I_B$):

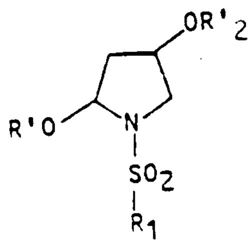

$(I_B)$

in which $R'_2$ has the values indicated previously for $R_2$, with the exception of a hydrogen atom, which compound of formula ($I_B$) is separated, if appropriate, into each of its isomers.

2. Process according to claim 1, characterized in that at the start a compound of formula (II) is used in which $R_1$ represents a phenyl radical.

3. Process according to claim 1 or 2, characterized in that at the start a compound of formula (II) is used in which R' represents a linear, branched or cyclic alkyl radical, containing up to 4 carbon atoms.

4. Process according to any one of claims 1 to 3, characterized in that compounds of formula (I) are prepared in which $R_2$ represents a hydrogen atom.

5. Process according to any one of claims 1 to 4, characterized in that 1-benzene-sulphonyl-2-oxo-3-hydroxy-5-ethoxy pyrrolidine is prepared.

6. Preparation process for pharmaceutical compositions, characterized in that at least one of the products of formula (I), as defined in claim 1, is used as active ingredient in a form intended for this use.

7. Preparation process for pharmaceutical compositions, characterized in that at least one of the products of formula (I), as defined in any one of claims 2 to 4, is used as active ingredient in a form intended for this use.

8. Preparation process for pharmaceutical compositions, characterized in that benzenesulphonyl-2-oxo-3-hydroxy-5-ethoxy pyrrolidine is used as active ingredient in a form intended for this use.

**Patentansprüche**
**Patentansprüche für folgende Vertragsstaaten : CH, DE, FR, GB, IT, LI, NL**

1. Verbindungen der allgemeinen Formel (I)

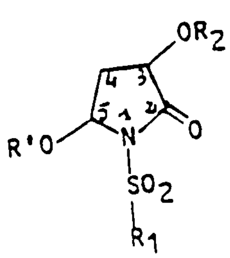

(I)

worin R' und $R_2$, die gleich oder voneinander verschieden sind, ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest darstellen, $R_1$ einen Phenyl- oder Naphthylrest, einen Thienyl-, Furyl-, Pyranyl-, Pyridyl-, Benzofuranyl-, Isobenzofuranyl, Chromanyl-, Isochromanyl, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest $R_1$ gegebenenfalls durch einen oder mehrere Substituenten substituiert sein kann, ausgewählt unter dem Hydroxylrest, dem Acetoxyrest, dem Methoxyrest oder

dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, dem Phenylrest und den Alkylsulfonylgruppen mit 1 bis 6 Kohlenstoffatomen,

**2.** Verbindungen der Formel (I) gemäß Anspruch 1, worin $R_2$ für ein Wasserstoffatom steht.

**3.** Verbindungen der Formel (I) gemäß Anspruch 1 oder 2, worin $R_1$ für einen Phenylrest steht.

**4.** Verbindungen der Formel (I) gemäß einem der Ansprüche 1 bis 3, worin R' für einen linearen, verzweigten oder cyclischen Alkylrest mit bis zu 4 Kohlenstoffatomen steht.

**5.** Produkt gemäß Anspruch 1 mit der folgenden Bezeichnung: 1-Benzolsulfonyl-2-oxo-3-hydroxy-5-ethoxy-pyrrolidin.

**6.** Als Arzneimittel die Verbindungen der Formel (I), wie in einem der Ansprüche 1 bis 4 definiert.

**7.** Als Arzneimittel die Verbindung gemäß Anspruch 5.

**8.** Pharmazeutische Zusammensetzungen, enthaltend als Wirkstoff zumindest ein Arzneimittel gemäß Anspruch 6 oder 7.

**9.** Verfahren zur Herstellung der Verbindungen der Formel (I) gemäß Anspruch 1, dadurch gekennzeichnet, daß man eine Verbindung der Formel (II)

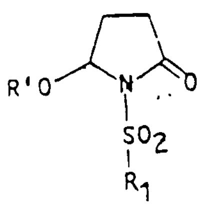

(II)

worin $R_1$ und R' die vorstehend angegebene Bedeutung besitzen, der Einwirkung eines Hydroxylierungsmittels unterzieht, um zu der entsprechenden Verbindung der Formel ($I_A$)

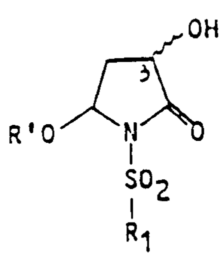

($I_A$)

in Form eines Gemisches der 3-Isomeren zu gelangen, welches man gewünschtenfalls in ein jedes der Isomeren auftrennt, wonach man gewünschtenfalls das Gemisch der Isomeren ($I_A$) oder ein jedes seiner Bestandteile der Einwirkung eines Veretherungs- oder Veresterungsmittels unterzieht, um die Verbindung der Formel ($I_B$)

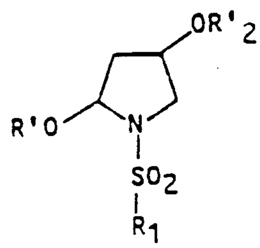

$(I_B)$

zu erhalten, worin $R'_2$ die vorstehend für $R_2$ angegebenen Bedeutungen mit Ausnahme derjenigen eines Wasserstoffatoms besitzt, welche Verbindung der Formel $(I_B)$ man gegebenenfalls in ein jedes seiner Isomeren trennt.

**Patentansprüche für folgenden Vertragsstaat : ES**

**1.** Verfahren zur Herstellung der Verbindungen der allgemeinen Formel

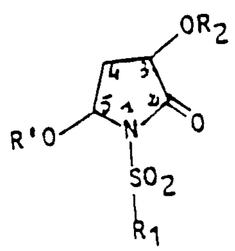

(I)

worin R' und $R_2$, die gleich oder voneinander verschieden sind, ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest darstellen, $R_1$ einen Phenyl- oder Naphthylrest, einen Thienyl-, Furyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest $R_1$ gegebenenfalls durch einen oder mehrere Substituenten substituiert sein kann, ausgewählt unter dem Hydroxylrest, dem Acetoxyrest, dem Methoxyrest oder dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, dem Phenylrest und den Alkylsulfonylgruppen mit 1 bis 6 Kohlenstoffatomen, dadurch **gekennzeichnet,** daß man eine Verbindung der Formel (II)

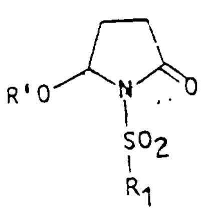

(II)

worin $R_1$ und R' die vorstehend angegebene Bedeutung besitzen, der Einwirkung eines Hydroxylierungsmittels unterzieht, um zu der entsprechenden Verbindung der Formel $(I_A)$

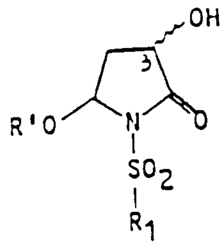

$(I_A)$

in Form eines Gemisches der 3-Isomeren zu gelangen, welches man gewünschtenfalls in ein jedes der Isomeren auftrennt, wonach man gewünschtenfalls das Gemisch der Isomeren ($I_A$) oder ein jedes seiner Bestandteile der Einwirkung eines Veretherungs- oder Veresterungsmittels unterzieht, um die Verbindung der Formel ($I_B$)

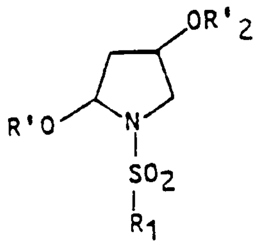

$(I_B)$

zu erhalten, worin $R'_2$ die vorstehend für $R_2$ angegebenen Bedeutungen mit Ausnahme derjenigen eines Wasserstoffatoms besitzt, welche Verbindung der Formel ($I_B$) man gegebenenfalls in ein jedes seiner Isomeren trennt.

2. Verfahren gemäß Anspruch 1, dadurch gekennzeichnet, daß man von einer Verbindung der Formel (II) ausgeht, worin $R_1$ für einen Phenylrest steht.

3. Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit bis zu 4 Kohlenstoffatomen steht.

4. Verfahren gemäß einem der Ansprüche 1 bis 3, dadurch gekennzeichnet, daß man Verbindungen der Formel (I) herstellt, worin $R_2$ ein Wasserstoffatom bedeutet.

5. Verfahren gemäß einem der Ansprüche 1 bis 4, dadurch gekennzeichnet, daß man 1-Benzolsulfonyl-2-oxo-3-hydroxy-5-ethoxypyrrolidin herstellt.

**Patentansprüche für folgenden Vertragsstaat : GR**

1. Verfahren zur Herstellung der Verbindungen der allgemeinen Formel

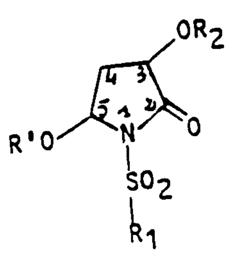

(I)

worin R' und $R_2$, die gleich oder voneinander verschieden sind, ein Wasserstoffatom, einen linearen, verzweigten oder cyclischen Alkylrest mit bis zu 8 Kohlenstoffatomen, einen Alkenylrest mit 2 bis 8

Kohlenstoffatomen, einen Acylrest mit 1 bis 6 Kohlenstoffatomen oder einen Benzyl- oder Phenethylrest darstellen, $R_1$ einen Phenyl- oder Naphthylrest, einen Thienyl-, Furyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei der Rest $R_1$ gegebenenfalls durch einen oder mehrere Substituenten substituiert sein kann, ausgewählt unter dem Hydroxylrest, dem Acetoxyrest, dem Methoxyrest oder dem Benzyloxyrest, den gesättigten oder ungesättigten, linearen, verzweigten oder cyclischen Alkylresten mit bis zu 18 Kohlenstoffatomen, den Halogenatomen, den Gruppen $CF_3$, $SCF_3$, $OCF_3$, $NO_2$, $NH_2$ oder C≡N, dem Phenylrest und den Alkylsulfonylgruppen mit 1 bis 6 Kohlenstoffatomen, dadurch **gekennzeichnet,** daß man eine Verbindung der Formel (II)

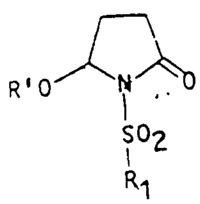

(II)

worin $R_1$ und R' die vorstehend angegebene Bedeutung besitzen, der Einwirkung eines Hydroxylierungsmittels unterzieht, um zu der entsprechenden Verbindung der Formel ($I_A$)

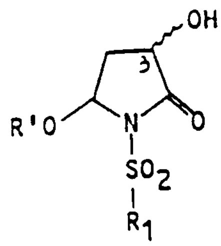

($I_A$)

in Form eines Gemisches der 3-Isomeren zu gelangen, welches man gewünschtenfalls in ein jedes der Isomeren auftrennt, wonach man gewünschtenfalls das Gemisch der Isomeren ($I_A$) oder ein jedes seiner Bestandteile der Einwirkung eines Veretherungs- oder Veresterungsmittels unterzieht, um die Verbindung der Formel ($I_B$)

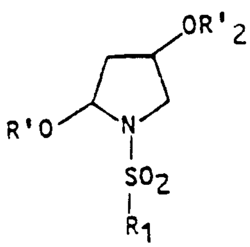

($I_B$)

zu erhalten, worin $R'_2$ die vorstehend für $R_2$ angegebenen Bedeutungen mit Ausnahme derjenigen eines Wasserstoffatoms besitzt, welche Verbindung der Formel ($I_B$) man gegebenenfalls in ein jedes seiner Isomeren trennt.

2. Verfahren gemäß Anspruch 1, dadurch gekennzeichnet, daß man von einer Verbindung der Formel (II) ausgeht, worin $R_1$ für einen Phenylrest steht.

3. Verfahren gemäß Anspruch 1 oder 2, dadurch gekenn zeichnet, daß man von einer Verbindung der Formel (II) ausgeht, worin R' für einen linearen, verzweigten oder cyclischen Alkylrest mit bis zu 4 Kohlenstoffatomen steht.

4. Verfahren gemäß einem der Ansprüche 1 bis 3, dadurch gekennzeichnet, daß man Verbindungen der Formel (I) herstellt, worin $R_2$ ein Wasserstoffatom bedeutet.

5. Verfahren gemäß einem der Ansprüche 1 bis 4, dadurch gekennzeichnet, daß man 1-Benzolsulfonyl-2-oxo-3-hydroxy-5-ethoxypyrrolidin herstellt.

6. Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung vorgesehene Form überführt.

7. Verfahren zur Herstellung von pharmazeutischen Zusammensetzungen, dadurch gekennzeichnet, daß man als Wirkstoff zumindest eines der Produkte der Formel (I), wie in einem der Ansprüche 2 bis 4 definiert, in eine für diese Verwendung vorgesehene Form überführt.

8. Verfahren zur Herstellung von pharmazeutischen Zusammensetzungen, dadurch gekennzeichnet, daß man als Wirkstoff das Benzolsulfonyl-2-oxo-3-hydroxy-5-ethoxypyrrolidin in eine für diese Verwendung bestimmte Form überführt.